Europäisches Patentamt

European Patent Office

Office européen des brevets

(11) Veröffentlichungsnummer: **0 273 140**
**A1**

(12) **EUROPÄISCHE PATENTANMELDUNG**

(21) Anmeldenummer: **87116114.7**

(22) Anmeldetag: **02.11.87**

(51) Int. Cl.⁴: **A61B 8/06** , A61K 49/00 , G01L 11/00

(30) Priorität: **05.11.86 DE 3637926**

(43) Veröffentlichungstag der Anmeldung:
**06.07.88 Patentblatt 88/27**

(84) Benannte Vertragsstaaten:
**ES GR**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Schlief, Reinhard, Dr.**
**Neue Strasse 21**
**D-1000 Berlin 37(DE)**
Erfinder: **Poland, Hans, Dr.**
**Spirdingseestrasse 29**
**D-1000 Berlin 49(DE)**

(74) Vertreter: **Maikowski, Michael, Dipl.-Ing. Dr.**
**Xantener Strasse 10**
**D-1000 Berlin 15(DE)**

(54) **Ultraschall-Manometrieverfahren in einer Flüssigkeit mittels Mikrobläschen.**

(57) Zum Zweck der Manometrie werden in eine Flüssigkeit, in welcher ein wolkenartiges Gebilde von stabilisierten Mikrobläschen erzeugt wurde, Ultraschallimpulse eingestrahlt und zu einer Wechselwirkung mit den Mikrobläschen gebracht. Die Amplituden-und/oder Frequenzänderungen der Ultraschallstreusignale werden gegenüber den eingestrahlten und/oder vorher empfangenen Ultraschallimpulsen registriert und ausgewertet. Mittel zur Blutdruckmessung, die bei dem Manometrieverfahren verwendet werden können, werden beschrieben.

Fig.1

## Ultraschall-Manometrieverfahren in einer Flüssigkeit mittels Mikrobläschen

Die Erfindung betrifft ein Ultraschall-Manometrieverfahren nach dem Oberbegriff des Anspruchs 1.

In der Zeitschrift "IEE Transaction on Biomedical Engineering, BME-24, Nr. 2, März 1977" wird auf den Seiten 107 bis 110 ein nichtinvasives Verfahren zur Druckmessung im Herzen beschrieben, bei dem von der Resonanzstreuung des Ultraschalls durch Bläschen Gebrauch gemacht wird. In der Flüssigkeit, deren Druck gemessen werden soll, werden Bläschen gleichförmiger Größe eines Gases, wie beispielsweise Stickstoff oder eines Inertgases erzeugt. Diese Bläschen haben die gleiche Resonanzfrequenz. Es wurden breitbandige Ultraschallimpulse in die Flüssigkeit eingestrahlt und das Spektrum des durchgelassenen oder gestreuten Ultraschalls untersucht. Bei dieser Arbeitsweise wird von einer Theorie ausgegangen, der die Wechselwirkung von Ultraschall und Einzelbläschen zu Grunde liegt. Es wurde die Resonanzfrequenzverschiebung bei Druckänderungen festgestellt. Bei diesem Verfahren wurde die Resonanzverschiebung geeicht. Auf diese Weise konnte die Druckänderung in einer Herzkammer ermittelt werden.

Diese Meßtechnik, die auf der Wechselwirkung von Ultra schall und Einzelbläschen beruht, hat sich nicht bewährt, weil die kleinen Einzelblasen eine zu kurze Lebensdauer haben und größere Blasen, insbesondere bei Messungen im Menschen, zu gefährlich sind. Größere Blasen können zu Embolien führen.

Die bei dem in der vorstehend zitierten Literaturstelle beschriebenen Verfahren verwendeten Bläschen sind wegen ihrer Größe nicht lungengängig. Diese Bläschen sind nach einer venösen Injektion im arteriellen Bereich nicht mehr vorhanden. Aus diesem Grunde können nach venöser Injektion bei dem bekannten Verfahren der diastolische und der systolische Druck nur im rechten Vorhof und in der rechten Kammer, nicht aber im linken Vorhof und in der linken Kammer gemessen werden.

In dieser Literaturstelle wird noch darauf hingewiesen, daß die Herstellung gleichförmiger Mikrobläschen außerordentlich schwierig ist.

Der Erfindung liegt die Aufgabe zu Grunde, ein Ultraschall-Manometrieverfahren in einer Flüssigkeit zur Verfügung zu stellen, mit dem eindeutige und reproduzierbare Messungen sowohl von Druckänderungen als auch von Druckabsolutwerten in Röhrensystemen, insbesondere an beliebigen Stellen im menschlichen Körper, hier insbesondere an beliebigen Stellen im Herzen, durchgeführt werden können. Ausgehend von einem Verfahren der eingangs genannten Art, wird diese Aufgabe jeweils erfindungsgemäß aufgrund des Kennzeichens von Anspruch 1, Anspruch 2 oder Anspruch 3 gelöst.

Mit Vorteil werden bei dem erfindungsgemäßen Verfahren demnach keine einzelnen Gasbläschen, sondern ein Ensemble, das, Mikrobläschen, die stabilisiert sind, enthält, verwendet. Es wird in der Flüssigkeit ein wolkenartiges Gebilde aus Mikrobläschen erzeugt. Dabei haben die stabilisierten Mikrobläschen eine Lebensdauer, die weit über der physikalisch für eine reine Flüssigkeit berechneten liegt. Das Verhalten eines solchen Mikrobläschenensembles ist nicht mehr mittels bekannter Resonanz-und Streumodelle von Einzelbläschen zu beschreiben. Es werden wesentlich größere, reproduzierbare Nutzsignale erzeugt. Dadurch kann eine eindeutige und reproduzierbare Messung durchgeführt werden, was bisher bei Einzelblasen nicht möglich war.

Mit besonderem Vorteil können Ultraschallimpulse eingestrahlt werden, deren Frequenzen kleiner als die Resonanzfrequenzen der Mikrobläschen oder gleich diesen sind. Dann wird zur Messung von Druckänderungen die flüssigkeitsdruckbedingte Verschiebung der Resonanzabsorption zu niedrigeren oder höheren Frequenzen hin gemessen.

Ferner können mit Vorteil Mikrobläschen mit einem standardisierten, d. h. bekannten und reproduzierbaren Spektrum der Resonanzabsorption verwendet werden. Mittels der empfangenen Ultraschallimpulse kann die spektrale Absorption zur Feststellung des Absolutwertes des momentanen Flüssigkeitsdruckes ermittelt werden. Bei Verwendung von Mikrobläschen mit bekanntem oder standardi siertem Resonanzspektrum können mit Vorteil Ultraschallimpulse mit einem vorbestimmten Frequenzbereich eingestrahlt werden. Zur Auswertung können Frequenzbereiche registriert werden, die außerhalb des Frequenzbereichs der eingestrahlten Ultraschallimpulse liegen.

Zur Ausschaltung von Fehlersignalen können mit Vorteil Ultraschallimpulse eingestrahlt werden, deren Frequenzen in einem vorbestimmten Verhältnis zu den Resonanzfrequenzen der Mikrobläschen stehen. Dabei können insbesondere n-te harmonische oder subharmonische Frequenzen bezüglich der Resonanzfrequenzen verwendet werden. Mit Vorteil können bei diesen Verfahren Amplitudenspektren im Frequenzbereich oberhalb der Eigenfrequenzen der Mikrobläschen ausgewertet werden. In diesen Bereichen ist die Druckabhängigkeit der Echoamplituden überwiegend frequenzunabhängig.

Es kann vorteilhaft sein, Mikrobläschen mit Durchmessern im Bereich von 0,1 bis 500 um zu verwenden. Dabei können Ultraschallfrequenzen mit Frequenzen im Bereich von 800 KHz bis 5 MHz eingestrahlt werden.

Durch die Erfindung wird ein Verfahren geschaffen, bei dem sowohl die Amplitudenänderungen als auch die Frequenzänderungen von Ultraschallsignalen berücksichtigt werden, die in Wechselwirkung mit Mikrobläschen in einer Flüssigkeit getreten sind.

Durch die Erfindung werden ferner Mittel zur Blutdruckmessung bereitgestellt.

Ein bevorzugtes Mittel wird dadurch gebildet, daß in einer Flüssigkeit Feststoffpartikel, die frei von Mikrobläschen sind und vorwiegend Partikel enthalten, die eine Anzahl Gas gefüllter Hohlräume aufweisen, die mit der Oberfläche in Verbindung stehen und eine Anzahl Kerne für eine Mikrobläschenbildung, insbesondere fein zerteilte Glucose, Laktoglucose, Maltose oder Salz mit Partikelgrößen im Bereich von 1 bis 5 μm gelöst werden, wobei das Verhältnis der Maße der Partikel zum Gasvolumen der Hohlräume so gewählt wird, daß dieses ausreicht, um die Flüssigkeit in der die Feststoffpartikel gelöst sind, in dem Bereich, der die Mikrobläschen umgibt, bezüglich des Gases in den Hohlräumen zu übersättigen.

Eine injizierbare Suspension kann eine wäßrige Trägerflüssigkeit aufweisen, deren Viskosität wesentlich größer als die von Wasser ist. Wenn dieses Mittel zur Blutdruckmessung mittels des Ultraschall-Manometrieverfahrens verwendet wird, können mit Vorteil die Blutgefäße vollständig gefüllt werden. Die mit dem Mittel erzeugten, Mikrobläschen passieren auch Kapillaren der Lungen, wodurch eine intraarterielle Druckmessung nach peripher-venöser Injektion prinzipiell an nahezu jeder beliebigen Stelle des Blutgefäßsystems möglich wird.

In vorteilhafter Weise kann ein Mittel zur Blutdruckmessung mittels des Ultraschall-Manometrieverfahrens verwendet werden, welches Mikropartikel der Mischung von einer halbfesten oder flüssigen grenzflächenaktiven Substanz mit einem nicht-grenzflächenaktiven Feststoff in einem flüssigen Träger enthält.

Derartige erfindungsgemäße Mittel werden in der EP-OS 123 235, die als Mittel zur Blutdruckmessung verwendet werden können, beschrieben.

Mit Vorteil kann das Mittel Mikropartikel enthalten, die als halbfeste oder flüssige grenzflächenaktive Substanz Lecithine, Polyoxyethylenfettsäureester, Glycerinpolyethylenglykolrizinoleat, Polyoxyethylenpolyoxypropylen-Polymere, Saccharoseester, Xyloglyceride, ungesättigte ($C_4$-$C_{20}$)-Fettalkohole, ungesättigte ($C_4$-$C_{20}$)-Fettsäuren, Mono-, Di-und Triglyceride, Fettsäureester als Mikropartikel in einer Menge von 0,01 bis 10 Gewichtsprozent enthalten. Ferner ist es möglich, daß das Mittel Mikropartikel enthält, die als halbfesten oder flüssigen grenzflächenaktiven Stoff Butylstearat. Sojaölsaccharoseglycerid oder Polyethylenglykolsorbitanmonostearat in einer Konzentration von 0,01 bis 5 Gewichtsprozent, vorzugsweise 0,04 bis 1 Gewichtsprozent, enthalten. Als nichtgrenzflächenaktive Feststoffe kann das Mittel Cyclodextrine, Monosaccharide, Disaccharide, Trisaccharide, Polyole oder anorganische oder organische Salze mit einer Konzentration von 5 bis 50 Gewichtsprozent enthalten. Das Mittel kann ferner Mikropartikel enthalten, die als nichtgrenzflächenaktiven Feststoff Galaktose, Lactose oder a-Cyclodextrin in einer Konzentration von 5 bis 50 Gewichtsprozent, vorzugsweise von 9 bis 40 Gewichtsprozent, enthalten.

Mit besonderem Vorteil kann das Mittel als physiologisch verträglichen flüssigen Träger Wasser, physiologische Elektrolytlösung, die wäßrige Lösung von ein-oder mehrwertigen Alkoholen wie Glycerin, Polyethylenglycol oder Propylenglykolmethylester oder wäßrige Lösung eines Mono-oder Disaccharides enthalten. Als physiologisch verträglicher flüssiger Träger kann aber auch Wasser oder physiologische Kochsalzlösung in dem Mittel enthalten sein. Ferner ist vorgesehen, daß das Mittel Mikropartikel einer Mischung von Butylstearat und Galaktose in Wasser enthält sowie eine Mischung von Sojaölsaccharoseglycerid und Galaktose in Wasser oder Polyethylenglycolsorbitanmonostearat und Galaktose in physiologischer Kochsalzlösung enthält.

Überraschenderweise wurde weiter gefunden, daß ein erfindungsgemäßes Mittel, welches Mikropartikel von Maltose, Dextrose, Lactose oder Galaktose und Gasbläschen in einem flüssigen Träger enthält, der Wasser, eine physiologische Elektrolytlösung wie 0,9 %ige wäßrige Natriumchloridlösung, Ringer-Lösung oder Tyrode-Lösung oder eine wäßrige Lösung von Maltose, Dextrose, Lactose oder Galaktose ist, ohne Zusatz von viskositätserhöhenden Stoffen wie beispielsweise Propylenglykol eine präzise und gut reproduzierbare Blutdruckmessung blutdurchströmter Organe wie Herz und Adern ermöglicht.

Derartige erfindungsgemäße Mittel werden in der EP-OS 131 540, die als Mittel zur Blutdruckmessung verwendet werden können, beschrieben.

Dabei kann dieses Mittel Mikropartikel aus Lactose in biz zu 25 %iger (Gewichtsprozent) wäßriger Lactose-Lösung enthalten. Insbesondere können auch Mikropartikel aus Galaktose in bis zu 20 %iger wäßriger Galaktose-Lösung enthalten sein oder Mikropartikel aus Galaktose in Wasser.

Es wurde überraschenderweise festgestellt, daß durch Suspendieren von Mikropartikeln einer festen grenzflächenaktiven Substanz gegebenenfalls in Kombination mit Mikropartikeln eines nicht-grenzflächenaktiven Feststoffes in einer Trägerflüssigkeit Mikrobläschen in Form eines Ensembles erhalten werden, die nach Injektion in eine periphäre Vene Druckmessung in der arteriellen linken Herzkammer ermöglichen.

Derartige erfindungsgemäße Mittel werden in der EP-OS 122 624, die als Mittel zur Blutdruckmessung verwendet werden können, beschrieben.

Bei diesem Mittel sind als grenzflächenaktive Substanz für die Herstellung der Mikropartikel alle Stoffe geeignet, die in den angewandten Mengen physiologisch verträglich sind, d. h. die eine geringe Toxizität besitzen und/oder biologisch abbaubar sind und deren Schmelzpunkt größer als Raumtemperatur ist.

Insbesondere geeignet sind Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerin-polyethylengly koloxystearat, Glycerinpolyethylenglykolrhizinoleat, ethoxylierte Sojasterine, ethoxylierte Rizinusöle und deren hydrierte Derivate, Cholesterol, Polyoxethylenfettsäurestearate und Polyoxyethylenpolyoxypropylen-Polymere mit dem Molgewicht von 6800-8975, 13300 und 16250, Saccharoseester wie Zuckerester, beispielsweise Saccharosedipalmitat und Saccharosemonolaurat oder Saccharoseglyceride sowie Xyloglyceride, gesättigte oder ungesättigte ($C_4$-$C_{20}$)-Fettalkohole oder ($C_4$-$C_{20}$)-Fettsäuren oder deren Metallsalze, Polyoxyethylenfettsäureester, Mono-, Di-und Triglyceride, Sorbitanfettsäureester, Fettsäureester der Saccharose oder Fettsäureester wie Butylstearat und Ascorbylpalmitat, wobei Calciumstearat, die Saccharoseester der Laurinsäure, der Stearinsäure und der Palmitinsäure sowie Ascorbylpalmitat bevorzugt sind.

Als grenzflächenaktive Substanzen sind solche besonders gut geeignet, die in der Trägerflüssigkeit relativ schwer löslich und im Blut relativ leicht löslich sind. Durch diesen Sprung im Auflöseverhalten der grenzflächenaktiven Substanzen kann die Auflösung des festen Materials, das reich an eingeschlossener Luft ist, gesteuert werden.

Die grenzflächenaktive Substanz wird in eine Konzentration von 0,01 bis 5 Gewichtsprozent, vorzugsweise von 0,04 bis 0,5 Gewichtsprozent verwendet.

Falls erwünscht, können die Mikropartikel der grenzflächenaktiven Substanz mit Mikropartikeln eines physio logisch verträglichen kristallinen Feststoffes kombiniert werden. Mann kann dafür organische oder anorganische Stoffe verwenden, zum Beispiel Salze wie Natriumchlorid, Natriumcitrat, Natriumacetat oder Natriumtartrat, Monosaccharide wie Glucose, Fructose oder Galaktose, Disaccharide wie Saccharose, Lactose oder Maltose, Pentosen wie Arabinose, Xylose oder Ribose oder Cyclodextrine wie α-, β-oder γ-Cyclodextrin, wobei Galaktose, Lactose und α-Cyclodextrin bevorzugt sind. Sie sind in einer Konzentration von 5 bis 50 Gewichtsprozent, vorzugsweise von 9 bis 40 Gewichtsprozent, im erfindungsgemäßen Mittel enthalten.

Es liegt ferner im Rahmen der Erfindung, daß ein Mittel zur Blutdruckmessung eine flüssige Mischung zur Aufnahme und Stabilisierung von mit physiologisch verträglichem Gas gefüllten Gasbläschen ist, bestehend aus der Mischung von 0,01 % bis 10 % eines Tensides oder Tensidgemisches mit einer wäßrigen oder mit Wasser mischbaren Trägerflüssigkeit und der Mischung von 0,5 % bis 50 % einer viskositätserhöhenden Substanz oder eines Substanzgemisches in einer wäßrigen oder mit Wasser mischbaren Trägerflüssigkeit, wobei beide Mischungen getrennt oder vereinigt vorliegen.

Derartige erfindungsgemäße Mittel werden in der EP-PS 0077 752, die als Mittel zur Blutdruckmessung verwendet werden können, beschrieben.

Als Tenside sind sowohl nichtionogene als auch ionogene Tenside geeignet. Als nichtionogene Tenside seien ge nannt: Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerin-polyethylenglykoloxystearat, Glycerinpolyethylenglykolrhizinoleat, ethoxylierte Sojasterine, ethoxylierte Rizinusöle und deren hydrierte Derivate, Cholesterol, Polyoxyethylenpolyoxypropylen-Polymere, wobei Polyoxyethylenfettsäurestearate und Polyoxyethylenpolyoxypropylen-Polymere mit dem Molgewicht 6800-8975, 13300 und 16250 bevorzugt sind. Sämtliche Prozentangaben beziehen sich auf Gewichtsprozente.

Als ionogene Tenside kommen in Frage: Quarternäre Ammoniumbase, Natriumlaurylsulfat, Natriumdioctylsulfosuccinat.

Die Mittel-Lösung kann dabei 0,01 bis 10 % eines Tensides oder des Gemisches mehrerer Tenside enthalten, wobei der bevorzugte Gehalt 0,5 bis 5 % Tensid oder Tensidgemisch beträgt.

Als viskositätserhöhende Substanzen kommen in Frage Mono-oder Polysaccharide wie Glucose, Lävulose, Galaktose, Lactose, Sorbit, Mannit, Xylit, Saccharose oder Dextrane, Cyclodextrine, Hydroxyethylstärke und Polyole. Als Polyole werden verwendet Glycerin, Polyglykole, Inulin und 1,2-Propandiol. Zur Viskositäterhöhung können weiterhin benutzt werden Proteine, proteinähnliche Stoffe, Aminosäuren oder Blutersatzstoffe wie beispielsweise Plasmaproteine, Gelatine, Oxypolygelatine und Gelatinederivate oder deren Gemische.

Die Konzentration dieser genannten Stoffe in der Lösung kann 0,5 bis 50 % betragen, wobei die Höchstkonzentration auch vom gelösten Stoff abhängt. So können beispielsweise Glucose oder Lactose mit einer Konzentration von 0,5 bis 50 % verwendet werden, wogegen Gelatine eine bevorzugte Konzentration von 0,5 bis 2 % hat. Die Oxypolygelatine wird bevorzugt mit einer Konzentration von 0,5 bis 10 % eingesetzt.

Man kann auch Tenside verwenden, die gleichzeitig viskositätserhöhend wirken wie beispielsweise Polyoxyethylenpolyoxpropylen-Polymere mit dem Molekulargewicht von 4750 bis 16250.

In diesem Fall beträgt die Konzentration der Tenside mit viskositätserhöhender Wirkung 1 % bis 20 %, vorzugsweise 3 % bis 10 %. Das Tensid oder Tensidgemisch wird vorzugsweise in Gegenwart des viskositätserhöhenden Stoffes oder Stoffgemische in einer Trägerflüssigkeit gelöst. Als Trägerflüssigkeit kann Wasser verwendet werden oder wäßrige Lösungen, die physiologisch verträglich sind wie beispielsweise physiologische Elektrolytlösungen wie physiologische Kochsalzlösung, Ringerlösung oder die wäßrigen Lösung von Natriumchlorid, Kalziumchlorid, Natriumhydrogencarbonat, Natriumcitrat, Natriumazetat oder Natriumtartrat oder Salzlösungen, wie sie üblicherweise als Infusionslösungen verwendet werden.

Ausführungsbeispiele der Erfinding sollen in der folgenden Beschreibung unter Bezugnahme auf die Fig. der Zeichnung erläutert werden.

Es zeigen:

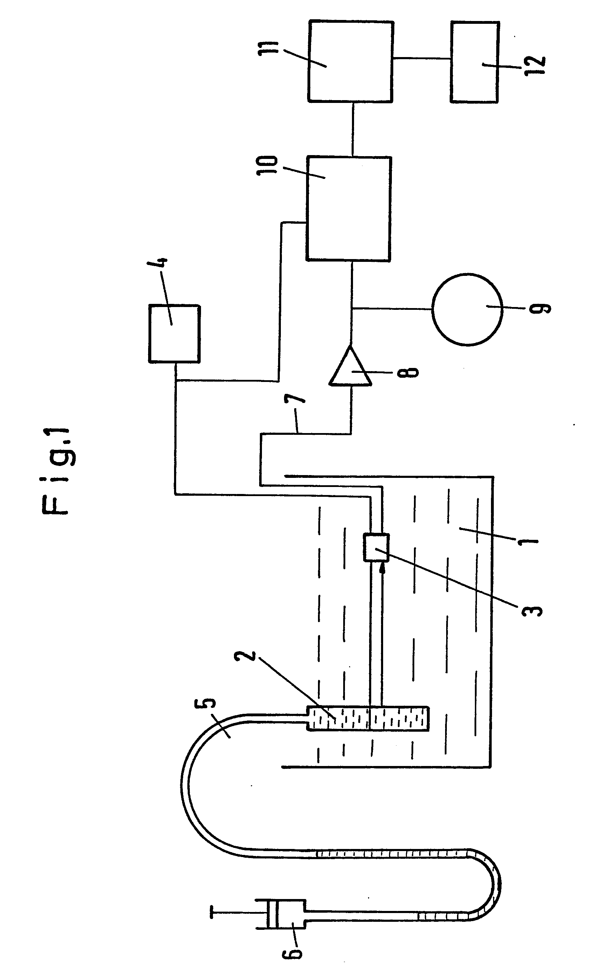

Fig. 1 eine schematische Darstellung eines Versuchsaufbaues zur Durchführung des Verfahrens,

Wie Fig. 1 zeigt, ist in einem Wasserbad 1 eine quaderförmige, doppelkammerige Plexiglasküvette 2 mit planparalleler Vorder-und Rückwand sowie ein Schallkopf 3 mit Sende-und Empfangsfunktion angeordnet. Der Schallkopf 3 steht mit einem Impulsgeber 4 in Verbindung. Für die Frequenzbereiche 800 KHz bis 2,2 MHz und 2,2 MHz bis 4,2 MHz wurden jeweils verschiedene Schallköpfe 3 mit entsprechend breitbandiger Impulscharakteristik benutzt. Wie dargestellt, erfolgte die Einstrahlung im 90 Winkel zur Vorderwand.

Die Plexiglasküvette 2 steht über eine Druckleitung 5 mit einem Druckgeber 6 in Verbindung.

Der Ausgang des Schallkopfes 3 ist über die Leitung 7 mit einem Verstärker 8 verbunden, dessen Ausgang, wie dargestellt, mit einem Oszillographen 9 und einer Komparatorschaltung 10 in Verbindung steht. Ein weiterer Eingang der Komparatorschaltung 10 ist, wie dargestellt, mit dem Impulsgeber 4 verbunden. Der Ausgang der Komparatorschaltung steht mit einem Laborrechner 11 in Verbindung, dessen Ausgang einen Drucker 12 speist.

Das Rückwandecho bzw. bei hoch konzentrierter Suspension, das vom Ensemble mit den Mikrobläschen zurückgestreute Signal wird verstärkt und oszillographisch sichtbar gemacht, sowie mit Hilfe eines Frequenzanalysators untersucht. Nach A/D-Wandlung wurden die Meßdaten in einem Laborrechner 11 gespeichert, so daß sich die Möglichkeit einer zusätzlichen Auswertung ergibt. Es können z. B. eine digitale Filterung oder eine graphische Darstellung vorgenommen werden.

## Ansprüche

1. Ultraschall-Manometrieverfahren in einer Flüssigkeit, bei dem Mikrobläschen in die Flüssigkeit eingebracht, in die Flüssigkeit Ultraschallimpulse eingestrahlt und nach Wechselwirkung mit den Mikrobläschen empfangen und ausgewertet werden,
dadurch gekennzeichnet, daß
in die Flüssigkeit ein Ensemble, bestehend aus den Mikrobläschen und Mikropartikeln aus einer Mischung aus einer halbfesten oder flüssigen grenzflächenaktiven Substanz mit einem nicht-grenzflächenaktiven Feststoff in einem flüssigen Träger, eingebracht wird.

2. Ultraschall-Manometrieverfahren in einer Flüssigkeit, bei dem Mikrobläschen in die Flüssigkeit eingebracht, in die Flüssigkeit Ultraschallimpulse eingestrahlt und nach Wechselwirkung mit den Mikrobläschen empfangen und ausgewertet werden,
dadurch gekennzeichnet, daß
in die Flüssigkeit ein Ensemble, bestehend aus den Mikrobläschen und Mikropartikeln einer festen, grenzflächenaktiven Substanz in Kombination mit Mikropartikeln eines nicht-grenzflächenaktiven Feststoffes in einem flüssigen Träger, eingebracht wird.

3. Ultraschall-Manometrieverfahren in einer Flüssigkeit, bei dem Mikrobläschen in die Flüssigkeit eingebracht, in die Flüssigkeit Ultraschallimpulse eingestrahlt und nach Wechselwirkung mit den Mikrobläschen empfangen und ausgewertet werden,
dadurch gekennzeichnet, daß
in die Flüssigkeit ein Ensemble, bestehend aus einer flüssigen Mischung zur Aufnahme und Stabilisierung von mit physiologisch verträglichem Gas gefüllten Gasbläschen, bestehend aus der Mischung von 0,01 % bis 10 % eines Tensides oder Tensidgemisches mit einer wäßrigen oder mit Wasser mischbaren Trägerflüssigkeit und der Mischung von 0,5 % bis 50 % einer viskositätserhöhenden Substanz oder eines Substanzgemisches in einer wäßrigen oder mit Wasser mischbaren Trägerflüssigkeit, eingebracht wird.

4. Verfahren nach einem der Ansprüche 1 - 3, dadurch gekennzeichnet, daß Ultraschallimpulse eingestrahlt werden, deren Frequenzen kleiner als die Resonanzfrequenzen der Mikrobläschen oder gleich diesen sind, und daß die Flüssigkeitsdruck bedingte Verschiebung der Resonanzabsorption zu niedrigeren oder höheren Frequenzen hin gemessen wird.

5. Verfahren nach einem der Ansprüche 1 - 4, dadurch gekennzeichnet, daß ein Ensemble mit einem standardisierten Spektrum der Resonanzabsorption verwendet wird, und daß mittels der empfangenen Ultraschallimpulse die spektrale Absorption zur Feststellung des Absolutwertes des momentanen Flüssigkeitsdruckes ermittelt wird.

6. Verfahren nach einem der Ansprüche 1 - 3, dadurch gekennzeichnet, daß ein Ensemble mit bekanntem oder standardisiertem Resonanzspektrum verwendet wird und Ultraschallimpulse mit einem vorbestimmten Frequenzbereich eingestrahlt werden und daß Frequenzbereiche registriert und ausgewertet werden, die außerhalb des Frequenzbereiches der eingestrahlten Ultraschallimpulse liegen.

7. Verfahren nach Anspruch 6, dadurch gekennzeichnet, daß Ultraschallimpulse eingestrahlt werden, deren Frequenzen in einem vorbestimmten Verhältnis, insbesondere, einem n-ten harmonischen oder subharmonischen, zu den Resonanzfrequenzen des Ensembles stehen.

8. Verfahren nach einem der Ansprüche 1 - 7, dadurch gekennzeichnet, daß Signalspektren im Frequenzbereich oberhalb der Eigenfrequenzen des Ensembles registriert und ausge wertet werden.

9. Verfahren nach einem der Ansprüche 1 - 8, dadurch gekennzeichnet, daß Mikrobläschen mit Durchmessern im Bereich von 0,1 bis 500 um verwendet werden.

10. Verfahren nach einem der Ansprüche 1 - 9, dadurch gekennzeichnet, daß Ultraschallimpulse mit Frequenzen im Bereich von 800 KHz bis 5 MHz eingestrahlt werden.

11. Mittel für eine Blutdruckmessung mittels des Ultraschall-Manometrieverfahrens nach einem der Ansprüche 1 oder 4 - 10, bestehend aus einer Suspension, die die Mikrobläschen und Mikropartikel, bestehend aus einer Mischung aus einer halbfesten oder flüssigen grenzflächenaktiven Substanz mit einem nicht-grenzflächenaktiven Feststoff in einem flüssigen Träger, enthält.

12. Mittel nach Anspruch 11, enthaltend als halbfeste oder flüssige grenzflächenaktive Substanz Lecithine, Polyoxyethylenfettsäureester, Glycerinpolyethylenglykolrizinoleat, Polyoxyethylenpolyoxypropylen-Polymere, Saccharoseester, Xyloglyceride, ungesättigte $(C_4-C_{20})$-Fettalkohole, ungesättigte $(C_4-C_{20})$-Fettsäuren, Mono-, Di und Triglyceride, Fettsäureester als Mikropartikel in einer Menge von 0,01 bis 10 Gewichtsprozent.

13. Mittel nach Anspruch 11 und 12, enthaltend als halbfeste oder flüssige grenzflächenaktiven Substanz Butylstearat, Sojaölsaccharoseglycerid oder Polyethylenglykolsorbitanmonostearat in einer Konzentration von 0,01 bis 5 Gwichtsprozent vorzugsweise 0,04 bis 1 Gewichtsprozent.

14. Mittel nach Anspruch 11, enthaltend als nicht-grenzflächenaktiven Feststoff Cyclodextrine, Monosaccharide, Disaccharide, Trisaccharide, Polyole oder anorganische oder organische Salze mit einer Konzentration von 5 bis 50 Gewichtsprozent.

15. Mittel nach Anspruch 11, enthaltend als nicht-grenzflächenaktiven Feststoff Galaktose, Lactose oder $\alpha$-Cyclodextrin in einer Konzentration von 5 bis 50 Gewichtsprozent, vorzugsweise von 9 bis 40 Gewichtsprozent.

16. Mittel nach Anspruch 11, enthaltend als flüssigen Träger, der physiologisch verträglich ist, Wasser, physiologische Elektrolytlösung, z. B. physiologische Kochsalzlösung, wäßrige Lösung von ein-oder mehrwertigen Alkoholen wie Glycerin, Polyethylenglykol oder Propylenglykolmethylester oder der wäßrigen Lösung eines Mono-oder Disaccharides.

17. Mittel nach Anspruch 11, enthaltend Mikropartikel einer Mischung aus Butylstearat und Galaktose in Wasser.

18. Mittel nach Anspruch 11, enthaltend Mikropartikel einer Mischung aus Sojaölsaccharose glycerid und Galactose in Wasser.

19. Mittel nach Anspruch 11, enthaltend Mikropartikel einer Mischung aus Polyethylenglykolsorbitanmonostearat und Galaktose in physiologischer Kochsalzlösung.

20. Mittel nach Anspruch 11, enthaltend Mikropartikel aus Maltose, Dextrose, Lactose oder Galaktose und Gasbläschen in einem flüssigen Träger wobei der flüssige Träger aus Wasser, physiologischer Elektrolytlösung wie 0,9 %iger Natriumchloridlösung, Ringer-Lösung oder Tyrode-Lösung oder einer wäßrigen Lösung von Maltose, Dextrose, Lactose oder Galaktose besteht.

21. Mittel nach Anspruch 20,
enthaltend
Mikropartikel aus Lactose in bis zu 25 %iger (Gewichtsprozent) wäßriger Lactose-Lösung.

22. Mittel nach Anspruch 20,
enthaltend
Mikropartikel aus Galaktose in Wasser, insbesondere in bis zu 20 %iger wäßriger Galaktose-Lösung.

23. Mittel für eine Blutdruckmessung mittels des Ultraschall-Manometrieverfahrens nach einem der Ansprüche 2 oder 4 - 10,
enthaltend
Mikrobläschen und Mikropartikel einer festen, grenzflächenaktiven Substanz in Kombination mit Mikropartikeln eines nicht-grenzflächenaktiven Feststoffes in einem flüssigen Träger.

24. Mittel nach Anspruch 23,
enthaltend
als feste grenzflächenaktive Substanz Lecithine, Polyoxyethylenfettsäureester, Glycerinpolyethylenglykolrizinoleat, Cholesterol, Polyoxyethylenpolyoxypropylen-Polymere, Saccharoseester, Xyloglyceride, gesättigte oder ungesättigte ($C_4$-$C_{20}$)-Fettalkohole, gesättigte oder ungesättigte ($C_4$-$C_{20}$)-Fettsäuren oder deren Metallsalze, Mono-, Di-und Triglyceride, Fettsäureester als Mikropartikel in einer Menge von 0,01 bis 10 Gewichtsprozent.

25. Mittel nach Anspruch 23,
enthaltend
Magnesiumstearat, Ascorbylpalmitat, Saccharosemonopalmitat, Saccharosemonstearat oder Saccharosedistearat als feste grenzflächenaktive Substanz in Form von Mikropartikel in einer Konzentration von 0,01 bis 5 Gewichtsprozent, vorzugsweise 0,04 bis 1 Gewichtsprozent.

26. Mittel nach Anspruch 23,
enthaltend
als gegebenenfalls vorhandene Mikropartikel eines nicht-grenzflächenaktiven Feststoffes Cyclodextrine, Monosaccharide, Disaccharide, Trisaccharide, Polyole oder anorganische oder organische Salze als Mikropartikel mit einer Konzentration von 5 bis 50 Gewichtsprozent.

27. Mittel nach einem der Ansprüche 23 - 26,
enthaltend
als gegebenenfalls vorhandenen nicht-grenzflächenaktiven Feststoff Galaktose, Lactose oder α-Cyclodextrin als Mikropartikel in einer Konzentration von 5 bis 50 Gewichtsprozent, vorzugsweise von 9 bis 40 Gewichtsprozent.

28. Mittel nach einem der Ansprüche 23 - 27,
enthaltend
als flüssigen Träger, der physiologisch verträglich ist, Wasser, physiologische Elektrolytlösung, wäßrige Lösung von ein-oder mehrwertigen Alkoholen wie Glycerin, Polyethylenglykol oder Propylenglykolmethylester oder wäßrige Lösung eines Mono-oder Disaccharides.

29. Mittel nach Anspruch 23 und 28,
enthaltend
als physiologisch verträglichen flüssigen Träger Wasser, physiologische Kochsalzlösung, 10 %ige wäßrige Lactose-Lösung oder 20 %ige wäßrige Galaktose-Lösung.

30. Mittel nach Anspruch 23,
enthaltend
Mikropartikel von Magnesiumstearat und von Galaktose in einer 20 %igen wäßrigen Galaktose-Lösung.

31. Mittel für eine Blutdruckmessung mittels des Ultraschall-Manometrieverfahrens nach einem der Ansprüche 3 oder 4 - 10,
bestehend
aus einer flüssigen Mischung zur Aufnahme und Stabilisierung von mit physiologisch verträglichem Gas gefüllten Gasbläschen, bestehend aus der Mischung von 0,01 % bis 10 % eines Tensides oder Tensidgemisches mit einer wäßrigen oder mit Wasser mischbaren Trägerflüssigkeit und der Mischung von 0,5 % bis 50 % einer viskositätserhöhenden Substanz oder eines Substanzgemisches in einer wäßrigen oder mit Wasser mischbaren Trägerflüssigkeit, wobei beide Mischungen getrennt oder vereinigt vorliegen.

32. Mittel nach Anspruch 31,
bestehend
aus der Mischung von 0,01 % bis 10 % eines Tensides oder Tensidgemisches in einer wäßrigen oder mit Wasser mischbaren Trägerflüssigkeit, die 0,05 bis 5 % eines physiologisch verträglichen carbonsauren Salzes enthält und der Mischung von 0,5 % bis 50 % einer viskositätserhöhenden Substanz oder eines Substanzgemisches mit einer wäßrigen oder mit Wasser mischbaren Trägerflüssigkeit, die die dem carbonsauren Salz äquivalente Menge physiologisch verträglicher Säure enthält.

33. Mittel nach Anspruch 31,
enthaltend
ein nichtionogenes Tensid, vorzugsweise ein Polyoxyethylenpolyoxypropylen-Polymeres.

34. Mittel nach Anspruch 33,
enthaltend
ein nichtionogenes Tensid, das aus Polyoxyethylenpolyoxypropylen-Polymere mit dem Molekulargewicht 6800 bis 8974 oder 16250 oder 13300 besteht.

35. Mittel nach Anspruch 33,
enthaltend
ein nichtionogenes Tensid, das aus einem Polyoxyethylenfettsäureester oder aus Polyoxyethylenstearaten besteht.

36. Mittel nach Anspruch 31, enthaltend ein ionogenes Tensid, vorzugsweise Natriumlaurylsulfat oder Natriumdioctylsulfosuccinat.

37. Mittel nach Anspruch 31, enthaltend als Trägerflüssigkeit Wasser oder mit Wasser mischbare ein-oder mehrwertige Alkohole, physiologische Elektrolytlösung oder eine Infusionslösung oder deren Gemische. ,

# Fig.1

0 273 140

Fa. Schering AG

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 265 251 (E.G. TICKNER) <br> * Zusammenfassung; Spalte 2, Zeile 55 - Spalte 4, Zeile 51; Spalte 6, Zeile 62 - Spalte 7, Zeile 8; Figur 1 * <br> --- | 1,3,9, 11,14, 15,26, 27 | A 61 B 8/06 <br> A 61 K 49/00 <br> G 01 L 11/00 |
| Y | EP-A-0 052 575 (ULTRA MED., INC.) <br> * Zusammenfassung; Seite 25, Zeilen 5-15; Seite 28, Zeilen 12-32; Seite 29, Zeile 1 - Seite 30, Zeile 17; Seite 31, Zeile 6 - Seite 32, Zeile 26; Seite 33, Zeilen 13-30; Seite 35 - Seite 38, Zeile 11; Seite 42, Zeilen 7-14 * | 1,3,9, 11,14, 15,26, 27 | |
| A | | 16,20, 28 | |
| D,A | EP-A-0 123 235 (SCHERING AG) <br> * Seite 4, Zeile 22 - Seite 7, Zeile 29 * <br> --- | 1-3,9, 11-16, 37 | |
| D,A | EP-A-0 077 752 (SCHERING AG) <br> * Seite 3, Zeilen 27-34; Seite 4, Zeilen 17-25; Seite 7, Zeile 10 - Seite 8, Zeile 31 * <br> --- | 1-3,11- 16,31- 37 | **RECHERCHIERTE SACHGEBIETE (Int. Cl.4)** <br><br> A 61 B <br> G 01 L |
| D,A | EP-A-0 122 624 (SCHERING AG) <br> * Seite 4, Zeile 22 - Seite 6, Zeile 30; Seite 8, Zeilen 1-16 * <br> --- | 1-3,11- 16,31- 37 | G 01 F |
| D,A | EP-A-0 131 540 (SCHERING AG) <br> --- <br> -/- | | |

Der vorliegende Recherchenbericht wurde für alle Patentansprüche erstellt

| Recherchenort | Abschlußdatum der Recherche | Prüfer |
|---|---|---|
| DEN HAAG | 11-02-1988 | RIEB K.D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

&: Mitglied der gleichen Patentfamilie, übereinstimmendes Dokument

EPO FORM 1503 03.82 (P0403)

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 640 271  (J.W. HORTON) <br> * Zusammenfassung; Spalte 1, Zeilen 30-56; Spalte 3, Zeilen 36-72; Spalte 6, Zeile 58 - Spalte 7, Zeile 9; Figur 1 * <br> --- | 1,4,6,9 ,28,29 | |
| A | EP-A-0 072 330  (FUJITSU LTD) <br> * Zusammenfassung; Seite 14, Zeilen 21-35; Seite 15, Zeilen 24-33; Figur 7 * <br> ----- | 1,6 | |
| | | | **RECHERCHIERTE SACHGEBIETE (Int. Cl.4)** |

Der vorliegende Recherchenbericht wurde für alle Patentansprüche erstellt

| Recherchenort | Abschlußdatum der Recherche | Prüfer |
|---|---|---|
| DEN HAAG | 11-02-1988 | RIEB K.D. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
   Dokument

EPO FORM 1503 03.82 (P0403)